## Europäisches Patentamt
## European Patent Office
## Office européen des brevets

(19)

(11) Veröffentlichungsnummer: **0 220 664**
**B1**

(12) **EUROPÄISCHE PATENTSCHRIFT**

(45) Veröffentlichungstag der Patentschrift:
**14.09.88**

(51) Int. Cl.⁴: **C 07 C 25/08, C 07 C 17/38**

(21) Anmeldenummer: **86114585.2**

(22) Anmeldetag: **21.10.86**

(54) **Verfahren zur Entfernung von m-Dichlorbenzol aus Dichlorbenzolgemischen.**

(30) Priorität: **30.10.85 DE 3538565**

(43) Veröffentlichungstag der Anmeldung:
**06.05.87 Patentblatt 87/19**

(45) Bekanntmachung des Hinweises auf die Patenterteilung:
**14.09.88 Patentblatt 88/37**

(84) Benannte Vertragsstaaten:
**DE FR GB**

(56) Entgegenhaltungen:
**DE - A - 1 468 004**
**US - A - 4 089 909**

(73) Patentinhaber: **BAYER AG, Konzernverwaltung RP Patentabteilung, D-5090 Leverkusen 1 Bayerwerk (DE)**

(72) Erfinder: **Röhlk, Kai, Dr., Odenthaler Marktweg 36, D-5060 Bergisch Gladbach 2 (DE)**
Erfinder: **Hartung, Sigurd, Dr., Neue Kempener Strasse 246, D-5000 Köln 60 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein Verfahren zur Entfernung von m-Dichlorbenzol aus Dichlorbenzolgemischen mit einem Gehalt von bis zu 35 Gew.-% an m-Dichlorbenzol, bezogen auf die Gesamtmenge an Dichlorbenzol im Gemisch, durch Chlorierung in flüssiger Phase bei erhöhter Temperatur in Gegenwart von Friedel-Crafts-Katalysatoren.

Aus der US-PS 4 089 909 ist ein Verfahren zur Trennung von Dichlorbenzolisomeren bekannt, bei dem in einem Dichlorbenzolgemisch vorzugsweise m-Dichlorbenzol zu 1,2,4-Trichlorbenzol und höheren Polychlorbenzolen mit elementarem Chlor in der flüssigen Phase in Gegenwart von Friedel-Crafts-Katalysatoren chloriert wird. Durch übliche fraktionierte Destillation und Kristallisation kann dann das verbleibende o-/p-Dichlorbenzol von höherchlorierten Chlorbenzolen getrennt werden.

Nachteilig bei diesem Trennverfahren ist, dass nicht nur das m-Dichlorbenzol im Gemisch chloriert wird, sondern zu einem beträchtigen Teil auch das wertvolle o- und p-Dichlorbenzol, wie die Tabellen der genannten US-PS zeigen.

Bei der Trennung und Reinigung der Dichlorbenzolisomeren nach dem in der US-PS 4 089 909 beschriebenen Verfahren sind also beträchtliche Verluste an gewünschtem o- und p-Dichlorbenzol unvermeidlich. Daher gestaltet sich dieses Verfahren wenig wirtschaftlich.

Aus der DE-A-1 4 68 004 ist bekannt, dass man Benzol und/oder chlorsubstituierte Benzole in die Dichlor- oder Tetrachlorstufe überführen kann, indem man das Benzol bzw. das chlorsubstituierte Benzol mit Chlor in Gegenwart eines Metallchlorid-Katalysators und unter Zusatz von Schwefel oder einer Schwefelverbindung behandelt. Diese Offenlegungsschrift enthält keinerlei Angaben darüber, dass m-Dichlorbenzol unter bestimmten Bedingungen wesentlich leichter chloriert werden kann als die anderen Dichlorbenzole, was die Basis für die vorliegende Erfindung darstellt.

Es wurde nun ein Verfahren zur Entfernung von m-Dichlorbenzol aus Dichlorbenzolgemischen mit einem Gehalt von bis zu 35 Gew.-% an m-Dichlorbenzol, bezogen auf die Gesamtmenge an Dichlorbenzol im Gemisch, durch Chlorierung in flüssiger Phase bei erhöhter Temperatur in Gegenwart von Friedel-Crafts-Katalysatoren gefunden, das dadurch gekennzeichnet ist, dass man die Chlorierung unter Zusatz von Schwefel, Schwefelverbindungen, Iod und/oder Iodverbindungen durchführt und anschliessend das Chlorierungsgemisch in üblicher Weise aufarbeitet.

In das erfindungsgemässe Verfahren können Dichlorbenzolgemische eingesetzt werden wie sie bei der Kernchlorierung von Benzol oder Chlorbenzol z.B. in flüssiger Phase in Gegenwart von Friedel-Crafts-Katalysatoren erhalten werden (vgl. Ullmanns Encyclopädie der technischen Chemie, Bd. 5, Seite 463 und Houben-Weyl, Methoden der organischen präparativen Chemie, Bd. 5, Seite 653). Solche Dichlorbenzolgemische enthalten je nach Herstellungsweise etwa 0,5 bis 3 Gew.-% an m-Dichlorbenzol sowie etwa 60 bis 70 Gew.-% an o- und p-Dichlorbenzol, wobei das ortho-/para-Verhältnis schwankt im Bereich von etwa 3,5:1 bis 1,5:1 (p-:o-Dichlorbenzol). Der Rest besteht aus unumgesetztem Benzol und Monochlorbenzol sowie aus höherchlorierten Chlorbenzolen.

Aus dem bei der Kernchlorierung erhaltenen Chlorierungsgemisch wird in üblicher Weise das Benzol und Chlorbenzol abdestilliert und gegebenenfalls das verbleibende Dichlorbenzolgemisch einer weiteren destillativen Trennung unterworfen. Je nach Anzahl der Trennschritte erhält man ein an m-Dichlorbenzol angereichertes Dichlorbenzolgemisch, das bis zu 35 Gew.-%, bevorzugt 0,3 bis 5 Gew.-%, besonders bevorzugt 1 bis 3 Gew.-% an m-Dichlorbenzol etwa 0,2 bis 40 Gew.-%, bevorzugt 25 bis 38 Gew.-% an o-Dichlorbenzol und etwa 50 bis 99 Gew.-%, bevorzugt 90 bis 98 Gew.-% an p-Dichlorbenzol enthält. Der Rest des Gemisches besteht aus Trichlorbenzolen und höherchlorierten Chlorbenzolen.

Das an m-Dichlorbenzol angereicherte Dichlorbenzolgemisch wird, wie zuvor beschrieben, in das erfindungsgemässe Verfahren eingesetzt.

Die Chlorierung des Dichlorbenzolgemisches wird üblicherweise bei Temperaturen von etwa 30 bis 120° C, bevorzugt 35 bis 70° C, durchgeführt.

Als Friedel-Crafts-Katalysatoren können beispielsweise eingesetzt werden: Eisen-III-chlorid, Aluminiumchlorid, Zinkchlorid und/oder Zinnchlorid, vorzugsweise Eisen-III-chlorid. Die Menge an einzusetzendem Friedel-Crafts-Katalysator ist nicht kritisch und beträgt üblicherweise weniger als 5 Gew.-%, bezogen auf die Dichlorbenzolmenge, bevorzugt 0,1 bis 4 Gew.-%.

Nach dem erfindungsgemässen Verfahren werden dem Friedel-Crafts-Katalysator Schwefel, Schwefelverbindungen, Iod und/oder Iodverbindungen zugegeben. Als Schwefelverbindungen seien genannt: Schwefelchloride, wie $S_2Cl_2$ Eisen-II-sulfid, Mercaptane und Thioether; als Iodverbindungen seien genannt: Alkyliodide sowie aromatische Iodverbindungen. Bevorzugt wird Schwefel, $S_2Cl_2$ und/oder Iod zugegeben.

Die Menge an Schwefel, Iod, Schwefelverbindungen und/oder Iodverbindungen, die dem Friedel-Crafts-Katalysator zugesetzt werden, beträgt üblicherweise etwa 0,01 bis 5 Gew.-%, bevorzugt 0,01 bis 1 Gew.-%, bezogen auf die Gesamtmenge an Dichlorbenzol im Gemisch.

Das Mengenverhältnis von Friedel-Crafts-Katalysator zu Iod oder Schwefel beträgt etwa 1:1 bis 5:1, bevorzugt 1,2:1 bis 3:1.

Die Chlorierung des Dichlorbenzolgemisches wird praktischerweise soweit fortgeführt bis der Gehalt an m-Dichlorbenzol im Gemisch unter etwa 0,05 Gew.-%, bevorzugt 0,02 Gew.-% beträgt. Es ist jedoch auch möglich die Chlorierung, falls dies erforderlich wird, soweit zu führen bis der Gehalt an m-Dichlorbenzol im Gemisch unter 0,005 Gew.-% beträgt. m-Dichlorbenzolgehalte von wesentlich über 0,05 Gew.-% im Dichlorbenzolgemisch sind nachteilig, da dadurch die Trennung der Dichlorbenzolisomeren erschwert wird.

Bei der erfindungsgemässen Chlorierung wird im allgemeinen ein geringer Überschuss an Chlor, bezogen auf die zu chlorierenden Aromaten, eingesetzt. Man setzt etwa 1,0 bis 1,5 Mol Chlor, bevorzugt 1,0 bis 1,05 Mol Chlor, pro Mol an zu chlorierenden Aromaten ein.

Nach Ende der Chlorierung kann das erhaltene Dichlorbenzolgemisch zur Trennung von o- und p-Dichlorbenzol fraktioniert destilliert werden.

Bei dem erfindungsgemässen Verfahren ist überraschend, dass durch den Zusatz von Schwefel, Iod, Schwefelverbindung und/oder Iodverbindungen bei der Chlorierung von an m-Dichlorbenzol angereicherten Dichlorbenzolgemischen praktisch nur das m-Dichlorbenzol zu höheren Chlorbenzolen chloriert wird und dass das o- und p-Dichlorbenzol unter diesen Chlorierungsbedingungen praktisch nicht weiterchloriert werden. Dadurch treten praktisch kaum Verluste an gewünschtem o- und p-Dichlorbenzol auf. Ausserdem reduziert sich durch den Zusatz von Schwefel, Iod, Schwefelverbindungen und/oder Iodverbindungen die Menge an einzusetzendem Chlor bei der Chlorierung des Gemisches.

Die nachfolgenden Beispiele sollen das erfindungsgemässe Verfahren verdeutlichen, ohne es jedoch auf diese Beispiele einzuschränken.

*Beispiel 1*

Einsatzgemisch:
95,0% para-Dichlorbenzol (p-DCIB)
4,5% meta-Dichlorbenzol (m-DCIB)
0,2% ortho-Dichlorbenzol (o-DCIB)
0,3% Trichlorbenzol, Benzol und Monochlorbenzol

| Temp. °C | Katalysator Gew.-% | Cl$_2$-Verbr. % | p-DCIB % | m-DCIB % | o-DCIB % | TriCIB+höher chlor. Benzole % |
|---|---|---|---|---|---|---|
| 55 | 0,1 FeCl$_3$ (Vergleich) | 0 | 95,0 | 4,5 | 0,20 | 0,30 |
|  |  | 68 | 93,3 | 0,16 | 0,11 | 6,43 |
|  |  | 76 | 82,3 | 0,11 | 0,10 | 17,49 |
|  |  | 84 | 80,2 | 0,07 | 0,09 | 19,64 |
|  |  | 92 | 79,1 | 0,05 | 0,09 | 20,76 |
|  |  | 100 | 77,5 | 0,03 | 0,09 | 22,38 |
| 55 | 0,1 FeCl$_3$ + 0,1 Iod | 0 | 95,0 | 4,5 | 0,20 | 0,30 |
|  |  | 100 | 92,25 | 0,02 | 0,09 | 7,64 |
| 55 | 0,1 FeCl$_3$ + 0,05 Iod | 0 | 95,0 | 4,5 | 0,20 | 0,30 |
|  |  | 100 | 91,7 | 0,03 | 0,14 | 8,13 |
| 55 | 0,1 FeCl$_3$ + 0,02 Schwefel | 0 | 95,0 | 4,5 | 0,20 | 0,30 |
|  |  | 100 | 94,2 | 0,03 | 0,13 | 5,64 |

Das Beispiel zeigt, dass mit FeCl$_3$ ohne den Zusatz von Iod oder Schwefel p- und o-Dichlorbenzol merklich bei der Chlorierung verbraucht werden.

*Beispiel 2*
Einsatzgemisch:
73,75% p-DCIB

25,40% o-DCIB
0,35% m-DCIB
0,50% Trichlorbenzol (TriCIB)

| Temp. °C | Katalysator Gew.-% | Cl$_2$-Verbr. % | p-DCIB % | m-DCIB % | o-DCIB % | TriCIB+höher chlor. Benzole % |
|---|---|---|---|---|---|---|
| 50 | 0,1 FeCl$_3$ (Vergleich) | 0 | 73,75 | 0,35 | 25,40 | 0,50 |
|  |  | 25 | 71,00 | 0,29 | 24,40 | 4,31 |
|  |  | 62 | 69,40 | 0,14 | 21,60 | 8,86 |
|  |  | 100 | 66,40 | 0,04 | 17,50 | 16,06 |
| 75 | 0,1 FeCl$_3$ + (Vergleich) | 0 | 73,75 | 0,35 | 25,40 | 0,50 |
|  |  | 20 | 70,90 | 0,33 | 23,60 | 5,17 |
|  |  | 40 | 69,60 | 0,24 | 21,70 | 8,46 |
|  |  | 100 | 64,40 | 0,06 | 15,30 | 20,24 |

| Temp. °C | Katalysator Gew.-% | Cl₂-Verbr. % | p-DClB % | m-DClB % | o-DClB % | TriClB+höher chlor. Benzole % |
|---|---|---|---|---|---|---|
| 50 | 0,1 FeCl₃ + 0,02 Schwefel | 0 50 75 100 | 73,75 73,40 73,30 73,00 | 0,35 0,100 0,050 0,025 | 25,40 24,40 23,70 23,00 | 0,5 2,1 2,95 3,9 |
| 50 | 0,1 FeCl₃ + 0,1 Iod | 0 33 66 100 | 73,75 72,65 71,50 70,90 | 0,35 0,18 0,06 0,023 | 25,40 23,30 20,70 19,10 | 0,50 3,9 7,7 10,0 |

*Beispiel 3*

Einsatzgemisch:
55,0% p-DClB

35,5% o-DClB
2,8% m-DClB
6,7% Trichlorbenzol

| Temp. °C | Katalysator Gew.-% | Cl₂-Verbr. % | p-DClB % | m-DClB % | o-DClB % | TriClB+höher chlor. Benzole % |
|---|---|---|---|---|---|---|
| 50 | 0,1 FeCl₃ | 0 100 | 55,0 43,8 | 2,8 0,03 | 35,5 13,3 | 6,7 42,87 |
| 35 | 0,1 FeCl₃ + 0,08 Schwefel | 0 50 100 | 55,0 54,5 54,4 | 2,8 0,2 0,02 | 35,5 32,5 30,5 | 6,7 12,80 15,08 |
| 50 | 0,02 FeCl₃ + 0,008 Schwefel | 0 100 | 55,0 52,4 | 2,8 0,02 | 35,5 26,7 | 6,7 20,88 |
| 50 | 0,1 FeCl₃ + 0,1 Iod | 0 50 100 | 55,0 53,2 52,4 | 2,8 0,5 0,02 | 35,5 29,0 21,2 | 6,7 17,30 26,38 |

*Beispiel 4*

Die Chlorierung wurde entsprechend der Tabelle II der US-PS 4 089 909 bei 66° C durchgeführt. Das Einsatzgemisch entsprach etwa dem in Tabelle II der US-PS aufgeführten Gemisch. Es setzte sich zusammen aus: 0,14 Gew.-% Benzol, 2,062 Gew.-% Monochlorbenzol, 35,701 Gew.-% m-Dichlorbenzol, 35,295 Gew.-% p-Dichlorbenzol und 26,787 Gew.-% o-Dichlorbenzol. Es wurden 1000 g des Gemisches chloriert.

*Zusammensetzung des Einsatzgemisches (Gew.-%)*

| Benzol | MClB | m-DClB | p-DClB | o-DClB | 1,2,4-TriClB | 1,2,3-TriClB | höher chlor. Benzole | |
|---|---|---|---|---|---|---|---|---|
| 0,14 | 2,062 | 35,701 | 35,295 | 26,787 | — | — | | |
| Beispiel | Katalysator | m-DClB (%) | p-DClB (%) | o-DClB (%) | 1,2,4-TriClB (%) | 1,2,3-TriClB (%) | höher chlor. Benzole (%) | eingeleitetes Chlor (g) |
| 1 ≙ US-PS 4 089 909 | FeCl₃ (0,1%) | 0,024 | 21,99 | 3,291 | 52,427 | 8,211 | 13,994 | 450 |

| Benzol | MCIB | m-DCIB | p-DCIB | o-DCIB | 1,2,4-TriCIB | 1,2,3-TriCIB | höher chlor. Benzole | |
|---|---|---|---|---|---|---|---|---|
| 0,14 | 2,062 | 35,701 | 35,295 | 26,787 | — | — | — | |
| Beispiel | Kataly-sator | m-DCIB (%) | p-DCIB (%) | o-DCIB (%) | 1,2,4-TriCIB (%) | 1,2,3-TriCIB (%) | höher chlor. Benzole (%) | eingeleitetes Chlor (g) |
| 2 | FeCl$_3$ (0,1%) + S (0,04%) | 0,024 | 34,997 | 20,09 | 39,618 | 3,578 | 1,655 | 245 |
| 3 | FeCl$_3$ (0,1%) + J$_2$ (0,1%) | 0,024 | 31,569 | 10,987 | 46,417 | 6,181 | 4,804 | 350 |

Das Beispiel zeigt, dass bei der Chlorierung mit FeCl$_3$ ohne Zusatz von Schwefel oder Jod p- und o-Dichlorbenzol merklich reagieren. Ausserdem ist ein wesentlich höherer Chlorüberschuss erforderlich.

## Patentansprüche

1. Verfahren zur Entfernung von m-Dichlorbenzol aus Dichlorbenzolgemischen mit einem Gehalt von bis zu 35 Gew.-% an m-Dichlorbenzol, bezogen auf die Gesamtmenge an Dichlorbenzol im Gemisch, durch Chlorierung in flüssiger Phase bei erhöhter Temperatur in Gegenwart von Friedel-Crafts-Katalysatoren, dadurch gekennzeichnet, dass man die Chlorierung unter Zusatz von Schwefel, Schwefelverbindungen, Iod und/oder Iodverbindungen durchführt und anschliessend das Chlorierungsgemisch in üblicher Weise aufarbeitet.

2. Verfahren nach Anspruch 1, dadurch gekennzeichnet, dass man Schwefel, Schwefelverbindungen, Iod und/oder Iodverbindungen in Mengen von 0,01 bis 5 Gew.-%, bezogen auf die Gesamtmenge an Dichlorbenzol im Gemisch, einsetzt.

3. Verfahren nach Ansprüchen 1 und 2, dadurch gekennzeichnet, dass das Mengenverhältnis von Friedel-Crafts-Katalysator zu Iod und/oder Schwefel 1:1 bis 5:1 beträgt.

## Claims

1. Process for the removal of m-dichlorobenzene from dichlorobenzene mixtures with an m-dichlorobenzene content of up to 35% by weight, relative to the total amount of dichlorobenzene in the mixture, by chlorination in the liquid phase at elevated temperature in the presence of Friedel-Crafts catalysts, characterized in that the chlorination is carried out with addition of sulphur, sulphur compounds, iodine and/or iodine compounds and the chlorination mixture is then worked up in the usual way.

2. Process as claimed in Claim 1, characterized in that sulphur, sulphur compounds, iodine and/or iodine compounds in amounts of 0.01 to 5% by weight, relative to the total amount of dichlorobenzene in the mixture, are used.

3. Process as claimed in Claims 1 and 2, characterized in that the ratio of the amount of Friedel-Crafts catalyst to the amount of iodine and/or sulphur is 1:1 to 5:1.

## Revendications

1. Procédé pour éliminer le m-dichlorobenzène de mélanges de dichlorobenzènes contenant jusqu'à 35% en poids de ce constituant par rapport aux dichlorobenzènes totaux contenus dans le mélange, par chloruration en phase liquide à chaud en présence de catalyseurs de Friedel-Crafts, caractérisé en ce que l'on procède à la chloruration avec adjonction de soufre, de composés du soufre, d'iode et/ou de composés de l'iode puis on traite le mélange de chloruration de la manière habituelle.

2. Procédé selon la revendication 1, caractérisé en ce que l'on utilise le soufre, les composés du soufre, l'iode et/ou les composés de l'iode en quantités de 0,01 à 5% en poids, par rapport à la quantité totale de dichlorobenzènes contenue dans le mélange.

3. Procédé selon les revendications 1 et 2, caractérisé en ce que les proportions relatives entre le catalyseur de Friedel-Crafts et l'iode et/ou le soufre sont de 1:1 à 5:1.